# EUROPEAN PATENT SPECIFICATION

(11) **EP 0 447 992 B1**
(45) Date of publication and mention of the grant of the patent: **01.03.1995**
(21) Application number: 91104061.6
(22) Date of filing: 15.03.1991
(51) Int. Cl.: B01D 45/14, G01N 33/00

(54) **Gas liquid separator**
Gasflüssigkeitsabscheider
Séparateur gaz-liquide

(30) Priority: 19.03.1990 JP 28553/90 U
(43) Date of publication of application: 25.09.1991
(73) Proprietor: HORIBA, LTD., Minami-ku Kyoto (JP)
(72) Inventor: Aoki, Junji, Sakyo-ku, Kyoto (JP); Koike, Hideki, Musimi-ku, Kyoto (JP); Sakamoto, Soji, Kila-Ward, Kyoto (JP)
(74) Representative: TER MEER STEINMEISTER & PARTNER GbR

(56) References cited:
- US-A- 4 678 488

## Description

The present invention relates to a gas liquid separator as claimed in the preamble of claim 1.

A gas liquid separator of this kind is already known from US-A-4 678 488. This known gas liquid separator comprising:
- a housing body having a gas liquid separating chamber;
- a centrifugal separator composed of a motor and a revolving disk driven by said motor, said revolving disk being positioned within said gas liquid separating chamber while keeping an appointed gap between its upper surface and the facing inner surface of the separating chamber, whereas the motor is positioned in a fitting hole formed in said housing body below said gas liquid separating chamber and opening downwards to the outside;
- a sample gas introducing conduit and a sample gas discharging conduit communicating with said gas liquid separating chamber, respectively, embouchures of both of said sample gas introducing and discharging conduits are placed in said gap, and the embouchure of said sample gas discharging conduit is disposed on the central axis of said revolving disk; and
- a liquid conduit leading from said gas liquid separating chamber to a drain pot for discharging a liquid fraction centrifugally separated by means of said centrifugal separator.

Said known gas liquid separator can be used as an auxiliary device of an infrared analyzer measuring, for example, an exhaust gas from a motor vehicle to analyze air pollutants. That is to say, in order to analyze an exhaust gas from a motor vehicle for measuring concentrations of C0₂, NOₓ, HC and the like contained in said exhaust gas, in general the infrared analyzer has to be supplied with a part of the exhaust gas as a sample gas.

Of course, in the case where the infrared analyzer is used to analyze the exhaust gas, if a liquid fraction is included in said sample gas, an error is produced in analytical results, so that a highly efficient gas liquid separator is necessary.

In the gas liquid separator having the above described construction, the sample gas is introduced into the gas liquid separating chamber through the sample gas introducing conduit by connecting a connecting portion on the side of the sample gas introducing conduit with the side of an engine of a motor vehicle through a pipe and connecting a connecting portion of the sample gas discharging conduit with the side of an infrared analyzer through a pipe. This sample gas is subjected to a gas liquid separation by means of the revolving disk revolved in a high speed and the sample gas, from which almost all of the liquid fraction has been removed, is guided to the side of said infrared analyzer through the sample gas discharging conduit. On the other hand, the liquid fraction is housed in a drain pot provided at the end of the liquid conduit communicated with said gas liquid separation chamber.

However, the sample gas in the gas liquid separator is exhausted from an engine of a motor vehicle and the like, so that an inside of the gas liquid separating chamber, the revolving disk 54 of said centrifugal separator and the like are apt to be soiled and damaged. In particular if impurities and foreign matters are accumulated in said gap a change in size of the gap (g) may occur, which means that the gas liquid separating characteristics are deteriorated, so that it is necessary to wash the inside of the gas liquid separating chamber and the like according to circumstances.

In this respect, a block member provided with said sample gas conduits must be separated from the housing body during the time when the washing is being carried out, and thus it is necessary to disconnect and to connect the pipes from and with the connecting portion on the side of the sample gas introducing conduit of the separated block member and the connecting portion on the side of the sample gas discharging conduit respectively. These operations of removing and connecting the pipes, however, are remarkably troublesome.

In addition, it is necessary to revolve the revolving disk of the centrifugal separator in a remarkably high speed (for example 11,000 to 13,000 rpm), so that the motor is remarkably damaged and thus a maintenance or exchange of the motor is necessary. But in the gas liquid separator having the above described conventional construction, the motor and the revolving disc are buried in the housing body, so that said maintenance or exchange is also troublesome.

### SUMMARY OF THE INVENTION

The present invention has been achieved paying attention to the above described matters and it is an object thereof to provide a gas liquid separator of the kind mentioned above allowing a more convenient maintenance of the centrifugal separator without requiring the removal and connection of the pipes with the housing body when the gas liquid separating chamber and the like are washed.

In order to achieve the above described object, the present invention has the following construction:
- the sample gas introducing conduit and the sample gas discharging conduit are formed in the wall of said housing body;
- the centrifugal separator is mounted on a motor block; and
- said motor block is sealing inserted into said fitting hole such that the centrifugal separator as a whole can simply be taken out from said housing body by removing the motor block from said housing body.

In the gas liquid separator having the above described construction, the sample gas, which has been introduced into the gas liquid separating chamber through the sample gas introducing conduit, is subjected to the gas liquid separation by means of the revolving disk revolving at a high speed and thus the sample gas, from which almost all of the liquid fraction have been removed, is guided to the side of the analyzer through the sample gas discharging conduit. On the other hand, the liquid fraction is discharged from the liquid conduit outlet through the liquid conduit.

Further, when the inside of the gas liquid separating chamber is washed, the centrifugal separator can be simply taken out of the housing body by removing the motor block from the housing body. In addition, when the centrifugal separator is removed, it is quite unnecessary to remove the pipes connected with the sample gas introducing conduit and the sample gas discharging conduit, respectively.

### BRIEF DESCRIPTION OF THE DRAWINGS

Fig. 1 is a sectional view showing a gas liquid separator according to one preferred embodiment of the present invention; and
Fig. 2 is a sectional view showing a gas liquid separator according to another preferred embodiment of the present invention.

### DETAILED DESCRIPTION OF THE PREFERRED EMBODIMENTS

The preferred embodiments will be below described with reference to the drawings.

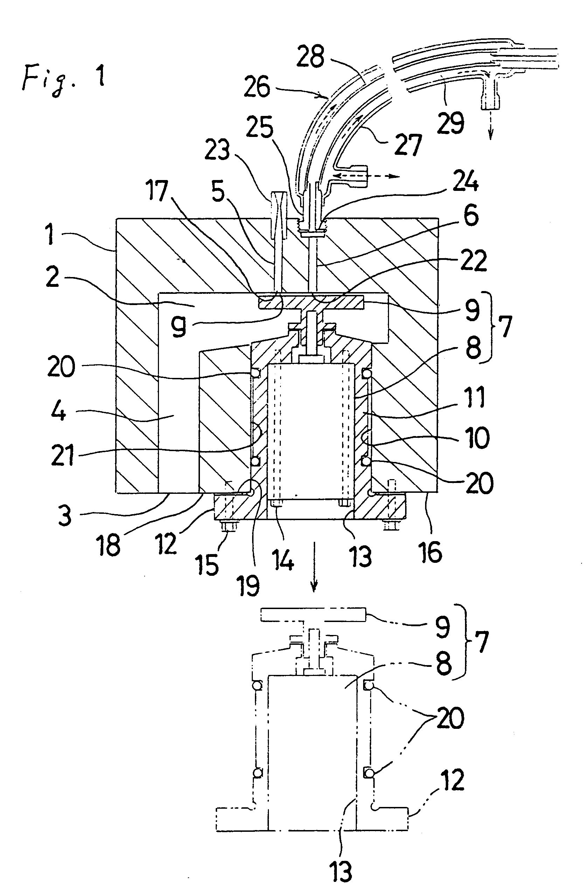

Referring to Fig. 1 showing the gas liquid separator according to one preferred embodiment of the present invention, reference numeral 1 designates a housing body made of, for example, aluminum which is provided with a gas liquid separating chamber 2 formed of a nearly rectangular hollow chamber and a liquid conduit 4 connected with said gas liquid separating chamber 2 at one end thereof and having a liquid conduit outlet 3 at the other end formed therewithin. Said housing body 1 is provided with a sample gas introducing conduit 5 and a sample gas discharging conduit 6 opened into outside at one end and opened into the gas liquid separating chamber 2 at the other end, respectively.

Reference numeral 7 designates a centrifugal separator provided below said gas liquid separating chamber 2. Said centrifugal separator 7 comprises a motor 8 and a revolving disk 9 revolved in a high speed by driving by means of said motor 8 and is detachably mounted on the housing body 1. That is to say, the housing body 1 is provided with a fitting hole 10 communicating therewith and opened into outside below the gas liquid separating chamber 2. On the other hand, the motor 8 is held in a motor block 11 fittedly inserted into said fitting hole 10. Said motor block 11 is made of, for example, aluminum and is provided with a flange member 12 on the lower end side thereof and a hole 13 opened into the lower end side. The motor 8 is mounted on the motor block 11 by means of screws 14 or fixedly adhered by the use of an epoxy resin without using said screw 14 under the condition that it is housed in said hole 13. And, the motor block 11 with the motor 8 held therein is inserted into the fitting hole 10 of the housing body 1 and said flange member 12 is fixedly mounted on a lower end surface 16 of the housing body 1 by means of screws 15 to mount the centrifugal separator 7 on the housing body 1. In this case, a gap (g) between said revolving disk 9 and an opening 17 on the inner side of said sample gas introducing conduit 5 is defined by a shoulder portion 18 of the housing body 1 and a shoulder portion 19 of the motor block 11. In addition, reference numeral 20 designates a sealing member provided between an outer circumferential surface of the motor block 11 and a wall surface 21 of the fitting hole 10. Furthermore, reference numeral 22 designates an opening on the inner side of said sample gas discharging conduit 6.

A choke 23, such as Venturi tube, is provided on the outer side of the sample gas introducing conduit 5 for lightening an influence by a pressure of a sample gas from a sample gas supply source (not shown). Besides, a filter 24 is provided on the outer side of the sample gas discharging conduit 6 for removing foreign matters which can not be separated by means of the centrifugal separator 7. A semipermeable dehumidifier 26 is connected with a connecting portion 25 provided outside of said filter 24. Said semipermeable dehumidifier 26 has a double tube structure. An inner tube 28 formed of a semipermeable membrane is provided within an outer tube 27 of the semipermeable dehumidifier 26 so that a concentration of moisture in said sample gas flowing through said inner tube 28 may be still more reduced by flowing a dry air or nitrogen gas through a passage 29 between said outer tube 21 and the inner tube 28.

In addition, although it is not shown in Fig. 1, a pipe communicating with said sample gas supply source is connected with said choke 23 and a pipe communicating with an analyzer, such as infrared analyzer, is connected with the semipermeable dehumidifier 26. And, a drain pot is connected with said liquid conduit outlet 3.

In the gas liquid separator having the above described construction, the sample gas, which has been introduced into the gas liquid separating chamber 2 through the sample gas introducing conduit 5, is subjected to the gas liquid separation in the revolving disk 9 revolving in a high speed and the sample gas, from which almost all of the liquid fraction have been removed, is guided to the side of said analyzer through the sample gas discharging conduit 6. On the other hand, the liquid fraction is extracted from the liquid conduit outlet 3 through said liquid conduit 4 to be housed in said drain pot (not shown).

When the inside of the gas liquid separating chamber 2 is to be washed, as shown by an imaginary line in Fig. 1, the centrifugal separator 7 can be simply taken out of the housing body 1 by removing the motor block 11 from the housing body 1 and thus the centrifugal separator 7 can be easily inspected or exchanged. When the centrifugal separator 7 is to be removed, it is quite unnecessary to remove said pipes connected with the sample gas introducing conduit 5 and the sample gas discharging conduit 6, respectively, and thus the choke 23 can be provided in the sample gas introducing conduit 5 and the filter 24 and the semipermeable dehumidifier 26 can be provided in the sample gas discharging conduit 6 to still more improve the capacity of the gas liquid separator of this type. In addition, the above described members 23, 24, 26 can be fixedly integrated with the housing body 1, so that the gas liquid separator can be compactized in construction.

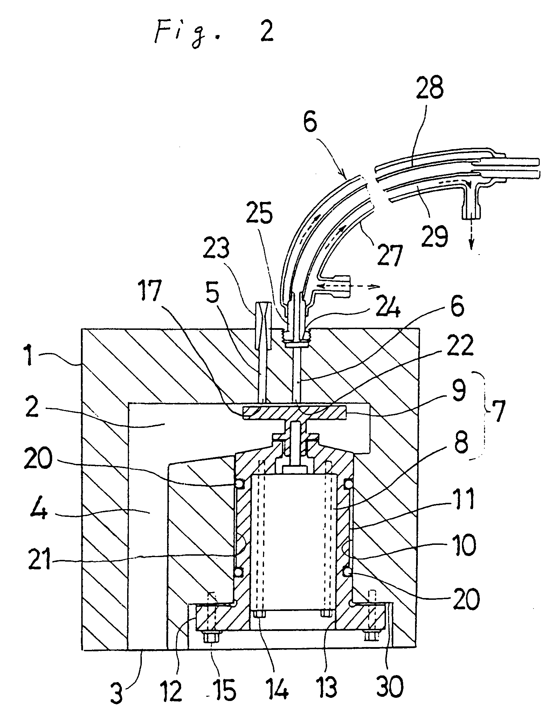

In another preferred embodiment of the present invention shown in Fig. 2, a fitting hole 10 is provided with a stepped portion 30 formed therein and a motor block 11 is completely housed in a housing body 1 so that a flange member 12 of said motor block 11 may be fixedly mounted on said stepped portion 30.

It goes without saying that also this construction can exhibit the same effects as in the above described first preferred embodiment.

As above described, according to the present invention, the housing body 1 is provided with the sample gas introducing conduit 5 and the sample gas discharging conduit 6 formed therein and the centrifugal separator 7 is detachably mounted on the housing body 1 through the motor block 11, so that it is unnecessary to remove and connect the pipes connected with the housing body 1 when the inside of the gas liquid separating chamber 2 and the like are to be washed and thus the maintenance of the centrifugal separator 7 can be easily achieved.

## Claims

1. A gas liquid separator comprising:
- a housing body (1) having a gas liquid separating chamber (2);
- a centrifugal separator (7) composed of a motor (8) and a revolving disk (9) driven by said motor (8), said revolving disk (9) being positioned within said gas liquid separating chamber (2) while keeping an appointed gap (g) between its upper surface and the facing inner surface of the separating chamber (2), whereas the motor is positioned in a fitting hole formed in said housing body (1) below said gas liquid separating chamber (2) and opening downwards to the outside;
- a sample gas introducing conduit (5) and a sample gas discharging conduit (6) communicating with said gas liquid separating chamber (2), respectively, embouchures (17, 22) of both of said sample gas introducing and discharging conduits (5, 6) are placed in said gap (g), and the embouchure (22) of said sample gas discharging conduit (6) is disposed on the central axis of said revolving disk (9); and
- a liquid conduit (4) leading from said gas liquid separating chamber (2) to a drain pot for discharging a liquid fraction centrifugally separated by means of said centrifugal separator (7) **characterized in that**
- the sample gas introducing conduit (5) and the sample gas discharging conduit (6) are formed in the wall of said housing body (1);
- the centrifugal separator (7) is mounted on a motor block (11); and
- said motor block (11) is sealingly inserted into said fitting hole (10) such that the centrifugal separator (7) as a whole can simply be taken out from said housing body (1) by removing the motor block (11) from said housing body (1).

2. The gas liquid separator as claimed in claim 1, **characterized in that** a Venturi tube is provided in the sample gas introducing conduit (5) and a filter (24) and a semipermeable dehumidifier (26) are provided in the sample gas discharging conduit (6).

3. The gas liquid separator as claimed in one of the claims 1 and 2, **characterized in that** the motor (8) is detachably mounted in a hole (13) of the motor block (11).

4. The gas liquid separator as claimed in claim 3, **characterized in that** the motor block (11) is provided with a flange (12) on the lower end side thereof.

5. The gas liquid separator as claimed in claim 4, **characterized in that** the housing body (1) is provided on its lower end side with a recess portion for receiving the flange (12) of the motor block (11).

6. The gas liquid separator as claimed in one of the claims 1 to 5, **characterized by** sealing means (20) on the outer circumferential surface of the motor block (11).

## Patentansprüche

1. Gas-Flüssigkeit-Trenneinrichtung mit:
- einem Gehäusekörper (1) mit einer Gas-Flüssigkeit-Trennkammer (2);
- einer Zentrifugaltrenneinrichtung (7) aus einem Motor (8) und einer vom Motor (8) angetriebenen umlaufenden Scheibe (9), die innerhalb der Gas-Flüssigkeit-Trennkammer (2) so angeordnet ist, daß sie zwischen ihrer Oberfläche und der zugewandten Innenfläche der Trennkammer (2) einen vorgegebenen Spalt (g) einhält, wohingegen der Motor in einem Befestigungsloch positioniert ist, das im Gehäusekörper (1) unter der Gas-Flüssigkeit-Trennkammer (2) ausgebildet ist und das sich nach unten zur Außenseite hin öffnet;
- einer Probengas-Einlaßleitung (5) und einer Probengas-Auslaßleitung (6), die jeweils mit der Gas-Flüssigkeit-Trennkammer (2) in Verbindung stehen, wobei die Mündungen (17, 22) sowohl der Probengas-Einlaß- als auch der -Auslaßleitung (5, 6) im genannten Spalt (g) liegen und die Mündung (22) der Probengas-Auslaßleitung (6) auf der Mittelachse der umlaufenden Scheibe (9) angeordnet ist; und
- einer Flüssigkeitsleitung (4), die von der Gas-Flüssigkeit-Trennkammer (2) her zu einem Ablaufgefäß zum Ausgeben des flüssigen Anteils, der durch Zentrifugieren durch die Zentrifugaltrenneinrichtung (7) abgetrennt wurde, führt; **dadurch gekennzeichnet, daß**
- die Probengas-Einlaßleitung (5) und die Probengas-Auslaßleitung (6) in der Wand des Gehäusekörpers (1) ausgebildet sind;
- die Zentrifugaltrenneinrichtung (7) auf einem Motorblock (11) angebracht ist; und
- der Motorblock (11) dicht in das Befestigungsloch (10) eingesetzt ist, so daß die Zentrifugaltrenneinrichtung (7) insgesamt einfach dadurch aus dem Gehäusekörper (1) herausgenommen werden kann, daß der Motorblock (11) dem Gehäusekörper (1) entnommen wird.

2. Gas-Flüssigkeit-Trenneinrichtung nach Anspruch 1, **dadurch gekennzeichnet, daß** ein Venturirohr in der Probengas-Einlaßleitung (5) vorhanden ist und ein Filter (24) und ein halbdurchlässiger Entfeuchter (26) in der Probengas-Auslaßleitung (6) vorhanden sind.

3. Gas-Flüssigkeit-Trenneinrichtung nach einem der Ansprüche 1 oder 2, **dadurch gekennzeichnet, daß** der Motor (8) wegnehmbar in einem Loch (13) des Motorblocks (11) befestigt ist.

4. Gas-Flüssigkeit-Trenneinrichtung nach Anspruch 3, **dadurch gekennzeichnet, daß** der Motorblock (11) an seiner unteren Endseite mit einem Flansch (12) versehen ist.

5. Gas-Flüssigkeit-Trenneinrichtung nach Anspruch 4, **dadurch gekennzeichnet, daß** der Gehäusekörper (1) an seiner unteren Endseite mit einem Aussparungsbereich zum Aufnehmen des Flanschs (12) des Motorblocks (11) versehen ist.

6. Gas-Flüssigkeit-Trenneinrichtung nach einem der Ansprüche 1 bis 5, **gekennzeichnet durch** eine Abdichtungseinrichtung (20) an der Außenumfangsfläche des Motorblocks (11).

## Revendications

1. Un séparateur gaz-liquide comprenant:
- un corps de boîtier (1) muni d'une chambre de séparation gaz-liquide (2);
- un séparateur centrifuge (7) composé d'un moteur (8) et d'un disque tournant (9) entraîné par ledit moteur (8), ledit disque tournant (9) étant positionné à l'intérieur de ladite chambre de séparation gaz-liquide (2) tout en maintenant un intervalle prédéterminé (g) entre sa surface supérieure et la surface inférieure qui lui fait face de la chambre de séparation (2), de telle manière que le moteur soit positionné dans un trou de montage formé dans ledit corps de boîtier (1) en-dessous de ladite chambre de séparation gaz-liquide (2) et s'ouvrant en bas vers l'extérieur;
- un conduit d'introduction d'échantillon de gaz (5) et un conduit de décharge d'échantillon de gaz (6) communiquant respectivement avec ladite chambre de séparation gaz-liquide (2), les embouchures (17, 22) des deux dits conduits d'introduction et de décharge d'échantillon de gaz (5, 6) étant placées dans ledit intervalle (g) et l'embouchure (22) dudit conduit de décharge d'échantillon de gaz (6) étant disposée sur l'axe central dudit disque tournant (9); et
- un conduit de liquide (4) conduisant de ladite chambre de séparation gaz-liquide (2) à un pot de drainage pour décharger une fraction liquide séparée par centrifugation au moyen dudit séparateur centrifuge (7), caractérisé en ce que le conduit d'introduction d'échantillon de gaz (5) et le conduit de décharge d'échantillon de gaz (6) sont formés dans la paroi dudit corps de boîtier (1);
- le séparateur centrifuge (7) est monté sur un bloc moteur (11); et
- ledit bloc moteur (11) est inséré de façon étanche dans ledit trou de montage (10) de telle façon que le séparateur centrifuge (7) puisse simplement être sorti comme un tout dudit corps de boîtier (1) en enlevant le bloc moteur (11) dudit corps de boîtier (1).

2. Le séparateur gaz-liquide tel que revendiqué à la revendication 1, caractérisé en ce qu'un tube de Venturi est prévu dans le conduit d'introduction d'échantillon de gaz (5) et en ce qu'un filtre (24) et un déshumidificateur semi-perméable (26) sont prévus dans le conduit de décharge d'échantillon de gaz (6).

3. Le séparateur gaz-liquide tel que revendiqué dans l'une des revendications 1 à 2, caractérisé en ce que le moteur (8) est monté de façon séparable dans un trou (13) du bloc moteur (11).

4. Le séparateur gaz-liquide tel que revendiqué dans la revendication 3, caractérisé en ce que le bloc moteur (11) est muni d'un flasque (12) sur sa face d'extrémité inférieure.

5. Le séparateur gaz-liquide tel que revendiqué dans la revendication 4, caractérisé en ce que le corps de boîtier (1) est muni sur sa face d'extrémité inférieure d'une partie en creux pour recevoir le flasque (12) du bloc moteur (11).

6. Le séparateur gaz-liquide tel que revendiqué dans l'une des revendications 1 à 5, caractérisé par des moyens d'étanchéité (20) sur la surface circonférentielle extérieure du bloc moteur (11).
